# EUROPÄISCHE PATENTSCHRIFT

(11) **EP 2 447 586 B1**
(45) Veröffentlichungstag und Bekanntmachung des Hinweises auf die Patenterteilung: **19.06.2013**
(21) Anmeldenummer: 11186778.4
(22) Anmeldetag: 26.10.2011
(51) Int. Cl.: F16L 19/10, F16L 33/22, F16L 47/04, F16L 19/065

(54) **Schlauchkupplung**
Hose coupling
Raccord pour tuyau

(30) Priorität: 26.10.2010 DE 102010049380
(43) Veröffentlichungstag der Anmeldung: 02.05.2012
(73) Patentinhaber: Meier, Michael, 67133 Maxdorf (DE)
(72) Erfinder: Meier, Michael, 67133 Maxdorf (DE)
(74) Vertreter: Thews, Karl

(56) Entgegenhaltungen:
- EP-A2- 0 034 493
- EP-A2- 0 879 980
- WO-A1-2011/027766
- DE-A1-102009 015 640
- GB-A- 672 453

## Beschreibung

Die Erfindung bezieht sich auf eine Schlauchkupplung aus Kunststoff bestehend aus einem Grundkörper und einem Verschlusskörper zum dichten Anschließen einer Schlauch-oder Rohrleitung, wobei der Grundkörper mindestens einen koaxial zu einer Mittelachse M im Grundkörper verlaufenden und in einem Rohrstutzen mit einer Mündung auslaufenden Kanal sowie einen koaxial zur Mittelachse M um den Rohrstutzen angeordneten Gehäuseabschnitt und einen optionalen, zwischen dem Kanal und dem Gehäuseabschnitt gebildeten, koaxial zur Mittelachse M angeordneten und zur Mündung hin offenen Ringspalt aufweist. Der Verschlusskörper weist eine koaxial zur Mittelachse M angeordnete Durchgangsöffnung zum Durchführen einer Leitung und ein zur manuellen Verschraubung ausgebildetes Bedienteil auf.

Der Grundkörper und der Verschlusskörper weisen jeweils zumindest eine in einer parallelen Richtung zur Mittelachse M wirkende Anschlagfläche auf und die beiden Anschlagflächen liegen nach dem Anschließen der Schlauch- oder Rohrleitung von außen sichtbar mittel- oder unmittelbar in einer Endlage E aneinander an.

Zwischen den beiden Anschlagflächen ist ein Spalt gebildet, der zumindest bis zum geschlossenen Zustand von Grundkörper und Verschlusskörper in einer Endlage E von außerhalb der Schlauchkupplung sichtbar ist. Der parallel zu den Anschlagflächen verlaufende Spalt hat den Vorteil, dass geringste Abweichungen im Bereich von wenigen Zehntel Millimetern vor der geschlossenen Position mit bloßem Auge erkannt werden können.

Es ist bereits eine Rohrverschraubung für die Befestigung von im Wesentlichen starren Schläuchen aus der DE 42 11 498 A1 bekannt, welche aus einem Verschlusskörper mit einer Einschuböffnung für den Schlauch sowie einer auf den Schlauch aufschiebbaren Dichtbuchse und einer auf den Verschlusskörper aufschraubbaren Überwurfmutter besteht. Der Verschlusskörper erweitert sich konisch in der Schlauchaufnahme und die Dichtbuchse weist einen Gegenkonus auf. Zwischen der Überowurfmuller und dem Dichteinsatz ist eine Sicherungsscheibe angeordnet, wobei die Sicherungsscheibe im auf den Schlauch geschobenen Zustand gegen die Auszugsrichtung des Schlauches geneigt ausgebildet ist und ggf. gegenüber dem Schlauch ein Untermaß aufweist, sodass beim Zug des Schlauches die Scheibe in das Material des Schlauches schneidet. Der Schlauch wird allein durch Klemmkräfte gesichert und hierbei elastisch und/oder plastisch verformt.

In der DE 32 25 172 A1 ist ein Anschlussnippel für Kunststoffrohre mit einem am einen Ende des Anschlussnippels angeordneten Konusring beschrieben, der einen Innenkonus aufweist. Zudem ist eine spannzangenartig ausgebildete, im Konusring verschiebbare Klemmhülse aus federndem Material vorgesehen, deren zum Nippel zeigendes Ende durch mehrere Längsschlitze in mehrere federnde Spannzangenarme aufgeteilt ist und deren freie Enden an ihrer dem Rohr zugekehrten Innenseite Haltekanten aufweisen. Die Haltekanten wirken mit ihren Außenseiten mit dem Innenkonus des Konusringes zusammen. Zwischen den freien Enden der Spannzangenarme und dem Nippel ist ein am Rohr anliegender O-Ring derart angeordnet, dass beim Einstecken des Rohres in die Klemmhülse die Spannzangenarme nach außen gespreizt werden, ihre Haltekanten unter Wirkung der Rückstellkraft der federnden Spannzangenarme in die Rohrwandung eindringen und das Rohr selbsttätig festhalten. Zum Lösen des Rohres ist der Konusring als eine auf den Anschlussnippel aufschraubbare Überwurfmutter ausgebildet, deren Betriebsstellung durch einen axialen Anschlag festgelegt ist.

Die DE 80 08 838 U1 verwirklicht ebenfalls das Klemmprinzip, ohne den Schlauch oder das Rohr zu verletzen. Hier ist ein Schlauchverbinder mit einem angeformten Rohrstutzen und Überwurfmuffen für Kunststoffschläuche, insbesondere für hydraulische und pneumatische Steuerleitungen, beschrieben. Der Rohrstutzen ist an seiner Basis im Abstand von etwa der Schlauchstärke von einem Ring mit Außengewinde umgeben, der einen Innenkonus aufweist. Die Überwurfmuffe ist mit einem mit dem Außengewinde zusammenwirkenden Innengewinde und einem zu diesem konzentrischen Innenring ausgebildet. Der Außendurchmesser des Innenrings ist kleiner als die Mündung des Innenkonus des den Rohrstutzen umgebenden Ringes.

In der DE 10 2009 015 640 A1, der dem Oberbegriff von Anspruch 1 offenbart, ist eine Vorrichtung zum Verbinden mit einem Ende einer röhrenartigen Leitung beschrieben, mit einem eine Längsachse aufweisenden Grundkörper und mit einem Klemmelement, mittels dessen die Leitung an der Vorrichtung lösbar fixierbar ist. Hierzu weist der Grundkörper einen Nippel auf, auf den die Leitung aufschiebbar und an dem die Leitung fixierbar ist. Die Vorrichtung weist eine definierte Endstellung für das Klemmelement beim Klemmen der Leitung auf, die auch im Falle einer weiteren Betätigung eines Betätigungselements der Vorrichtung beibehalten wird, so dass eine Überlastung des Klemmelements oder der Leitung verhindert ist. Hierzu können die Klemmzungen auf ihrer Außenfläche einen weiteren Abschnitt aufweisen, der in dem die Leitung klemmenden ausgelenkten Zustand der Klemmzungen im Wesentlichen zylindrisch zur Längsachse des Grundkörpers ausgerichtet ist, und der mit dem Betätigungselement derart zusammenwirkt, dass bei einem weiteren Betätigen des Betätigungselements die Klemmzungen radial nicht weiter in Richtung auf die Leitung auslenkbar sind. Dadurch werden die Klemmzungen und mithin das Klemmmittel nur in einem vorgebbaren Umfang in Richtung auf die Leitung ausgelenkt, so dass eine definierte Endposition der Klemmzungen in Bezug auf die Leitung gegeben ist. Es kann daher nicht zu einer Überbeanspruchung der Leitung kommen, die für eine dauerhafte Dichtwirkung nachteilig sein könnte.

In der GB 672 453 A ist eine Schlauchkupplung beschrieben, bei der die maximale Klemmkraft allein durch plastische Deformation und nicht durch einen Anschlag maximiert ist.

Der Erfindung liegt die Aufgabe zugrunde, eine Schlauchkupplung derart auszubilden und anzuordnen, dass eine präzise Zugfestigkeit der Verbindung an der Leitung ohne Einschneiden der Leitung und gleichzeitig eine einfache Herstellung der Schlauchkupplung erreicht wird.

Gelöst wird die Aufgabe erfindungsgemäß dadurch, dass der Verschlusskörper ein an die Durchgangsöffnung in axialer Richtun anschließendes welches je nach Ausführun in den Ringspalt zum Anschließen der Leitung einführbar und an die Leitung anlegbar ist, aufweist.

Zudem wird die Aufgabe erfindungsgemäß dadurch gelöst, dass das Maß der Klemmkraft des als Klemmhülse ausgebildeten Klemmelements erst dann maximal ist, wenn sich der Grundkörper und der Verschlusskörper in der Endlage E befinden, wobei die Klemmhülse eine in Umfangsrichtung linienförmigen Klemmefläche bildet. Hierdurch wird erreicht, dass das Maß, mit dem die Leitung durch die Klemmhülse im geschlossenen Zustand geklemmt wird, durch den Anschlag zwischen dem Grundkörper und dem Verschlusskörper definiert ist und gleichzeitig die richtige Position kontrolliert werden kann. Der Grundkörper und der Verschlusskörper müssen zum Anschließen der Leitung so weit in axialer Richtung bis zu einer axialen Endlage E geschlossen werden, dass die beiden Anschlagflächen mittel- oder unmittelbar aneinander anliegen. Ein zu weites Hineindrehen des Verschlusskörpers wird durch den Anschlag verhindert und eine nicht ausreichend feste Klemmposition wird durch einen nicht erreichten Anschlag signalisiert.

Ferner ist es vorteilhaft, dass der Gehäuseabschnitt eine zur Mündung hin im Durchmesser größer werdende und in den Ringspalt gerichtete und zur Mittelachse M koaxial angeordnete Konusfläche aufweist, die Klemmhülse eine umlaufende Gegenkonusfläche aufweist, wobei ein Winkel k der Konusfläche größer als ein Winkel g der Gegenkonusfläche ist. Dadurch wird erreicht, dass das vordere Ende der Klemmhülse beim Einführen in den Ringspalt stärker verjüngt wird als der übrige Teil der Klemmhülse. Damit werden eine fast linienförmige Dichtfläche und gleichzeitig eine fast linienförmige Deformationsfläche erreicht. Der relative Klemmdruck an der linienförmigen Anlagefläche ist dadurch wesentlich größer, als wenn die Klemmhülse mit ihrer gesamten Konusfläche am Ringspalt anliegen würde.

Hierzu ist es eine besondere Möglichkeit gemäß einer Weiterbildung, dass die die Einstecktiefe der Klemmhülse in den Ringspalt maximal ist, wenn sich der Grundkörper und der Verschlusskörper in der Endlage E befinden.

Erfindungsgemäß ist die Schlauchkupplung zweiteilig ausgebildet und die beiden Anschlagflächen in axialer Richtung unmittelbar aneinander anlegbar sind. Die beiden Bauteile werden in Anspruch 1 beschrieben. Ein erstes Bauteil bildet der Grundkörper mit dem Gehäuseabschnitt und dem Rohrstutzen. Das zweite Bauteil bildet der Verschlusskörper mit dem Bedienteil und der Klemmhülse. Die Leitung wird in den Grundkörper in den Ringspalt zwischen den Gehäuseabschnitt und dem Rohrstutzen eingesteckt und mit dem schon auf die Leitung aufgefädelten Verschlusskörper verschraubt und damit die Leitung angeschlossen.

Ferner ist es vorteilhaft, dass die Schlauchkupplung mit einem separaten Distanzring drei- oder mehrteilig ausgebildet ist und die beiden Anschlagflächen mittelbar über den Distanzring in axialer Richtung aneinander anlegbar sind. Neben den beiden vorstehend beschriebenen Bauteilen wird der Distanzring eingebracht, sodass die Anschlagposition früher erreicht wird. Dadurch wird das Maß der Klemmwirkung reduziert, weil die Klemmhülse nicht so weit in den Ringspalt eindringen kann. Dies ist insbesondere dann vorteilhaft, wenn die gleiche Schlauchkupplung für verschieden dimensionierte Leitungen verwendet werden soll. Durch den Distanzring können nämlich Leitungen mit gleichem Innendurchmesser und mit unterschiedlicher Wandstärke verschraubt werden.

Es ist auch vorgesehen, dass zumindest eine Anschlagfläche in Form mehrerer Teilflächen oder durch eine durchgehende Fläche ausgebildet ist, die in radialer Richtung um die Mittelachse M umlaufen. Die umlaufend nebeneinander angeordneten Teilflächen sind insbesondere vorteilhaft, um die Menge an Kunststoffmaterial zu reduzieren. Die Flächen bilden somit einen Spalt, der rechtwinklig beziehungsweise in radialer Richtung zur Mittelachse verläuft.

In besonders vorteilhafter Weise ist es vorgesehen, dass der Grundkörper und der Verschlusskörper jeweils ein koaxial zur Mittelachse M angeordnetes Gewinde aufweisen, wobei die beiden Gewinde zum Anschließen der Leitung miteinander verschraubt werden können. Durch ein Gewinde ist das Verschließen der Schlauchkupplung wesentlich präziser und mit mehr Klemmkraft möglich als mit einer anderen Verschlussmöglichkeit wie beispielsweise einem Klick- oder einem Bajonettverschluss.

Von besonderer Bedeutung ist für die vorliegende Erfindung, dass die Klemmhülse um ein Maß K durch die beiden Gewinde in axialer Richtung in den Ringspalt einführbar ist, um das der Grundkörper und der Verschlusskörper durch die Gewinde beim Zudrehen geschlossen werden. Dadurch wird die Montage vereinfacht, weil das für die Verschraubung notwendige Maß auf ein Minimum reduziert wird. Eine Schraubbewegung ist somit nicht notwendig, um die Klemmhülse in axialer Richtung vor dem Ringspalt zu positionieren. Diese Position wird durch das Ineinanderfügen von Grundkörper und Verschlusskörper erreicht.

Im Zusammenhang mit der erfindungsgemäßen Ausbildung und Anordnung ist es von Vorteil, dass die Klemmhülse beim Schließen der Schlauchkupplung so weit in den Ringspalt eingeschoben wird, dass die Leitung durch die Klemmhülse in radialer Richtung um ein Maß zwischen 4 und 6 % einer ursprünglichen Wandstärke deformiert wird. Aufgrund der Anschlagverhältnisse und bei entsprechender Dimensionierung der Bauteile sind solche Toleranzen erreichbar. Solange der Kunststoff der Leitung innerhalb dieser Toleranz gedehnt wird, wird die molekulare Struktur des Kunststoffs erhalten, welche maßgeblich zur Druckstabilität und zur Eignung für hohe Temperaturen beiträgt. Eine

Überdehnung des Kunststoffs führt zu molekularen Rissen und langfristig zur mangelnden Dichtheit, auch wenn die Leitung noch so fest geklemmt wird.

Vorteilhaft ist es ferner, dass als Außengewinde ausgebildete Gewinde in axialer Richtung an das Bedienteil und an das Gewinde in axialer Richtung die Klemmhülse anschließt. Dabei kann der Verschlusskörper einfacher ausgebildet werden, als wenn das Außengewinde am Grundkörper vorgesehen ist. Dabei ist es notwendig, dass das als Innengewinde ausgebildete Gewinde am Gehäuseabschnitt vorgesehen ist, wobei der Gehäuseabschnitt in axialer Richtung über den Rohrstutzen hervorsteht.

Vorteilhaft ist es hinsichtlich einer einfachen Herstellung und gleichzeitig einfachen Handhabung, dass das Bedienteil und die Klemmhülse einteilig und materialidentisch ausgebildet sind.

Weitere Vorteile und Einzelheiten der Erfindung sind in den Patentansprüchen und in der Beschreibung erläutert und in den Figuren dargestellt. Es zeigt:
- Figur 1: eine Explosionszeichung einer Schlauchkupplung mit einem Grundkörper, einem Verschlusskörper und einer Leitung;
- Figur 2: eine Detailansicht des Grundkörpers nach Fig. 1;
- Figur 3: eine Detailansicht des Verschlusskörpers nach Fig. 1;
- Figur 4: eine Ansicht des Grundkörpers mit fast ganz eingeführtem Verschlusskörper;
- Figur 5: eine Ansicht einer geschlossenen Schlauchkupplung;
- Figur 6: eine Draufsicht gemäß Ansicht nach Fig. 5;
- Figur 7: eine Schlauchkupplung mit einem separatem Distanzring;
- Figur 8: eine Schlauchkupplung mit einer separaten Klemmhülse, die nicht zum Erfindung gehört.

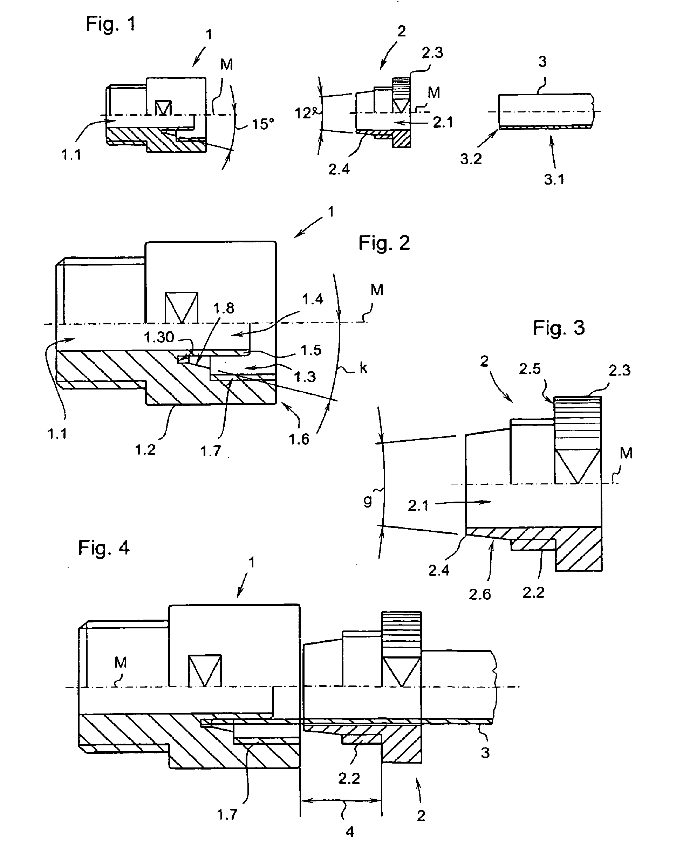

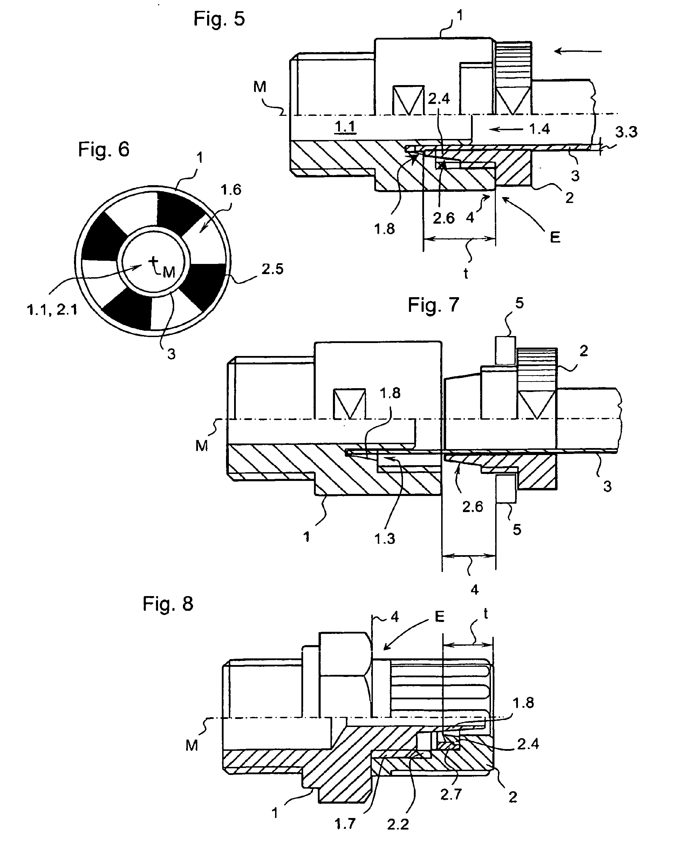

In Fig. 1 ist eine erfindungsgemäße Schlauchkupplung bestehend aus einem Grundkörper 1 und einem Verschlusskörper 2 in einer Explosionsansicht dargestellt. Die Schlauchkupplung ist rotationssymmetrisch zu einer Mittelachse M ausgebildet. Der Verschlusskörper 2 wird, wie in Fig. 4 und 5 näher dargestellt, über ein Ende 3.1 einer Leitung 3 gestülpt und die Leitung 3 mit dem Ende 3.1 in den Grundkörper 1 eingesteckt. Durch Verschrauben des Verschlusskörpers 2 mit dem Grundkörper 1 wird die Leitung 3 zwischen den beiden Körpern 1, 2 geklemmt und somit in Richtung der Mittelachse M, also in axialer Richtung, festgelegt und gleichzeitig abgedichtet. Eine geschlossene Schlauchkupplung mit einer angeschlossenen Leitung 3 ist in Fig. 5f dargestellt.

Fig. 2 zeigt im Detail, dass der Grundkörper 1 einen Kanal 1.1 aufweist, der durch den Grundkörper 1 in axialer Richtung zur Mittelachse M hindurchtritt. Innerhalb des Grundkörpers 1 ist ein Rohrstutzen 1.5 gebildet, der von einem Gehäuseabschnitt 1.2 umgeben ist, sodass der Gehäuseabschnitt 1.2 und der Rohrstutzen 1.5 einen Ringspalt 1.3 bilden.

Der Ringspalt 1.3 wird in Richtung nach innen durch den zylinderförmigen Rohrstutzen 1.5 gebildet, der eine in Richtung des Verschlusskörpers 2 ausgerichtete Mündung 1.4 aufweist, über die die Leitung 3 gestülpt wird. Nach außen wird der Ringspalt 1.3 durch ein als Innengewinde ausgebildetes Gewinde 1.7 und eine in axialer Richtung daran anschließende Konusfläche 1.8 gebildet. Das Gewinde 1.7 korrespondiert mit einem in Fig. 3 dargestellten Gewinde 2.2 des Verschlusskörpers 2.

In axialer Richtung ist der Ringspalt 1.3 durch eine an die Konusfläche 1.8 angrenzende Anschlagfläche 1.30 für die Leitung 3 begrenzt, welche in axialer Richtung an den Grundkörper 1 anstößt, wie in Fig. 4 dargestellt ist.

Ferner weist der Grundkörper 1 eine in axialer Richtung wirkende Anschlagfläche 1.6 auf, gegen die eine Anschlagfläche 2.5 des in Fig. 3 dargestellten Verschlusskörpers 2 beim Verschließen der Schlauchkupplung anschlägt.

Der Verschlusskörper 2 ist gemäß Fig. 3 aus einem Bedienteil 2.3 und einer an das Bedienteil 2.3 anschließenden Klemmhülse 2.4 gebildet, die an die Leitung 3 anlegbar ist. Zwischen der Klemmhülse 2.4 und dem Bedienteil 2.3 ist das Gewinde 2.2 umfänglich am Verschlusskörper 2 vorgesehen. In Fig. 3 ist die Anschlagfläche 2.5 dargestellt, die mit der Anschlagfläche 1.6 bis zum geschlossenen Zustand der Schlauchkupplung einen in Fig. 4 dargestellten Spalt 4 bildet und im geschlossenen Zustand an die Anschlagfläche 1.6 von außen sichtbar anschlägt. Durch eine mit dem Kanal 1.1 korrespondierende Durchgangsöffnung 2.1 wird die Leitung 3 weiter geführt.

Das Einfädeln des Verschlusskörpers 2 beziehungsweise der Klemmhülse 2.4 in den Ringspalt 1.3 wird durch die anzuschließende Leitung 3 erleichtert. In Fig. 4 ist erkennbar, dass beim Einfädeln die Klemmhülse 2.4 automatisch in den Ringspalt 1.3 geführt wird, bis die Gewinde 1.7, 2.2 aneinander anliegen. Durch das Einschrauben des Verschlusskörpers 2 in den Grundkörper 1 wird der Spalt 4 geschlossen und die notwendige Klemmkraft, jedoch nicht zu viel Klemmkraft zwischen der Klemmhülse 2.4 und der Leitung 3 sowie dem Rohrstutzen 1.5 erzeugt.

In Fig. 5 ist dargestellt, wie die Anschlagfläche 2.5 des Verschlusskörpers 2 nach dem Verschrauben der beiden Körper 1, 2 gegen die Anschlagfläche 1.6 des Grundkörpers 1 anschlägt, sodass eine weitere Verschraubung und eine erhöhte Klemmwirkung, nachdem der zwischen den beiden Körpern 1, 2 gebildete Spalt 4 geschlossen ist, nicht erhöht werden kann.

Es ist erkennbar, dass das Material der Leitung 3 im Bereich der Klemmhülse 2.4 und im Bereich des Rohrstutzens 1.5 deformiert wird, sodass eine Wandstärke 3.3 geringer ist als bei der übrigen Leitung 3. Das maximal erwünschte und durch die Klemmhülse 2.4 begrenzte Maß an Deformation ist durch die Materialeigenschaften der Leitung 3 vorgegeben. Die molekulare Struktur der Leitung 3 muss so weit erhalten bleiben, dass mit Kunststoffleitungen Drücke bis 30 bar und Temperaturen bis 250 °C bewerkstelligt werden können.

Die maximale Deformation soll nur in einem sehr kleinen Bereich der Leitung 3 erfolgen, sodass bevorzugt eine in Umfangsrichtung linienförmige Klemmfläche gebildet wird. Dies wird durch eine unterschiedliche Neigung der Konusfläche 1.8 und einer Gegenkonusfläche 2.6 erreicht. Die Gegenkonusfläche 2.6 ist mit einem Neigungswinkel k von ca. 6° (Fig. 1 und 3) flacher als die Konusfläche 1.8 mit einem Neigungswinkel g von ca. 15° (Fig. 1 und 2). Dadurch liegt die Klemmhülse 2.4 in Umfangsrichtung linienförmig an der Konusfläche 1.8 an und wird beim Schließen der Schlauchkupplung auch linienförmig gegen die Leitung 3 gedrückt.

Sobald der Spalt 4 zwischen dem Grundkörper 1 und dem Verschlusskörper 2 bzw. zwischen den beiden Anschlagflächen 1.6, 2.5 von außen sichtbar geschlossen ist, ist die Verschraubung ausreichend fest, sodass eine ausreichende Abdichtung und eine in axialer Richtung ausreichende Klemmwirkung für die Leitung 3 erreicht ist.

In Fig. 6 ist eine Ansicht eines Ausführungsbeispiels mit Blickrichtung in axialer Richtung auf den Verschlusskörper 2 dargestellt, bei dem das Bedienteil 2.3 und somit die vier Anschlagflächen 1.6 des Verschlusskörpers 2 aus mehreren Teilkörpern mit jeweils einer Teilfläche gebildet sind, die als Teilkreissegmente um die Mittelachse M angeordnet sind. Die Blickrichtung ist in Fig. 5 mit einem Pfeil verdeutlicht.

In Fig. 7 ist zusätzlich ein Distanzring 5 dargestellt, der in axialer Richtung zwischen den beiden Anschlagflächen 1.6, 2.5 positioniert und auf den Verschlusskörper 2 aufgesteckt ist. Durch den Distanzring 5 wird bei Verwendung von Leitungen 3 mit einer größeren Wandstärke 3.3 früher die notwendige Klemmwirkung erreicht, sodass der Spalt 4 früher geschlossen werden muss.

In Fig. 8 ist ein Schlauchkupplung dargestellt, die nicht zum Erfindung gehört, bei dem die Klemmhülse 2.4 als separates Bauteil ausgebildet ist und der Grundkörper 1 dabei keinen Ringspalt 1.3 bildet. Diese bei kleineren Durchmessern von Leitungen 3 angewandte Schlauchkupplung basiert jedoch auf dem gleichen Prinzip, nach dem die Klemmwirkung durch eine Anschlagposition der beiden Anschlagflächen 1.6, 2.5 in axialer Richtung begrenzt ist. Ferner wird in Fig. 8 deutlich, dass die gemäß Fig. 1 bis 7 beschriebene Schlauchkupplung auch mit einem Außengewinde 1.7 am Grundkörper 1 und einem Innengewinde 2.2 am Verschlusskörper 2 ausgebildet sein kann.

Die separate Klemmhülse 2.4 wird durch einen Lagerring 2.7 in radialer Richtung im Verschlusskörper 2 gelagert. Die linienförmige Anlage an die Leitung 3 wird durch die Innenfläche der Klemmhülse 2.4 erreicht, die in Bezug auf die Mittelachse M entgegen dem Ausführungsbeispiel nach Fig. 1 bis 7 nicht parallel, sondern angestellt ist.

In einem nicht dargestellten Ausführungsbeispiel weist der Grundkörper 1 ein als Außen- oder Innengewinde und der Verschlusskörper 2 ein korrespondierendes, als Innen- oder Außengewinde ausgebildetes Gewinde auf.

## Patentansprüche

1. Zweiteilige Schlauchkupplung aus Kunststoff bestehend aus einem Grundkörper (1) und einem Verschlusskörper (2) zum dichten Anschließen einer Schlauch- oder Rohrleitung (3), wobei der Grundkörper (1)
a) mindestens einen koaxial zu einer Mittelachse M im Grundkörper (1) verlaufenden und in einem Rohrstutzen (1.5) mit einer Mündung (1.4) auslaufenden Kanal (1.1) und
b) einen koaxial zur Mittelachse M um den Rohrstutzen (1.5) angeordneten Gehäuseabschnitt (1.2) und einen zwischen dem Kanal (1.1) und dem Gehäuseabschnitt (1.2) gebildeten, koaxial zur Mittelachse M angeordneten und zur Mündung (1.4) hin offenen Ringspalt (1.3) und der Verschlusskörper (2)
c) eine koaxial zur Mittelachse M angeordnete Durchgangsöffnung (2.1) zum Durchführen einer Leitung (3) und
d) ein zur manuellen Verschraubung ausgebildetes Bedienteil (2.3), wobei
**e)** der Grundkörper (1) und der Verschlusskörper (2) jeweils zumindest eine in einer parallelen Richtung zur Mittelachse M wirkende Anschlagfläche (1.6, 2.5) aufweisen und die beiden Anschlagflächen (1.6, 2.5) nach dem Anschließen der Schlauch- oder Rohrleitung (3) von außen sichtbar mittel- oder unmittelbar in einer Endlage E aneinander anliegen,
**dadurch gekennzeichnet, dass**
f) der Verschlusskörper (2) ein an die Durchgangsöffnung (2. 1) in axialer Richtung anschließende Klemm-element (2.4) **,** welches in den Ringspalt (1.3) zum Anschließen der Leitung (3) einführbar und an die Leitung (3) anlegbar ist, aufweist,
g) das Maß der Klemmkraft des als Klemmhülse (2.4) ausgebildeten Klemmelements erst dann maximal ist, wenn sich der Grundkörper (1) und der Verschlusskörper (2) S. in der Endlage E befinden, und
h) die Klemmhülse (2.4) eine in Umfangsrichtung linienförmigen Klemmfläche bildet.

2. Schlauchkupplung nach Anspruch 1, **dadurch gekennzeichnet, dass** der Gehäuseabschnitt (1.2) eine zur Mündung (1.4) hin im Durchmesser größer werdende und in den Ringspalt (1.3) gerichtete und zur Mittelachse M koaxial angeordnete Konusfläche (1.8) aufweist, die Klemmhülse (2.4) eine umlaufende Gegenkonusfläche (2.6) aufweist, wobei ein Winkel k der Konusfläche (1.8) größer als ein Winkel g der Gegenkonusfläche (2.6) ist.

3. Schlauchkupplung nach Anspruch 2, **dadurch gekennzeichnet, dass** die Einstecktiefe (t) der Klemmhülse (2.4) in den Ringspalt (1.3) maximal ist, wenn sich der Grundkörper (1) und der Verschlusskörper (2) in der Endlage E befinden.

4. Schlauchkupplung nach einem der vorhergehenden Ansprüche, **dadurch gekennzeichnet, dass** die beiden Anschlagflächen (1.6, 2.5) in axialer Richtung unmittelbar aneinander anlegbar sind.

5. Schlauchkupplung nach einem der vorstehenden Ansprüche, **dadurch gekennzeichnet, dass** die Schlauchkupplung mit einem separaten Distanzring (5) drei- oder mehrteilig ausgebildet ist und die beiden Anschlagflächen (1.6, 2.5) mittelbar über den Distanzring (5) in axialer Richtung aneinander anlegbar sind.

6. Schlauchkupplung nach einem der vorhergehenden Ansprüche, **dadurch gekennzeichnet, dass** der Grundkörper (1) und der Verschlusskörper (2) jeweils ein koaxial zur Mittelachse M angeordnetes Gewinde (1.7, 2.2) aufweisen, wobei die beiden Gewinde (1.7, 2.2) zum Anschließen der Leitung (3) miteinander verschraubt werden können.

7. Schlauchkupplung nach Anspruch 6, **dadurch gekennzeichnet, dass** die Klemmhülse (2.4) um ein Maß durch die beiden Gewinde (1.7, 2.2) in axialer Richtung in den Ringspalt (1.3) einführbar ist, um das der Grundkörper (1) und der Verschlusskörper (2) durch die Gewinde (1.7, 2.2) beim Zudrehen geschlossen werden.

8. Schlauchkupplung nach einem der vorstehenden Ansprüche, **dadurch gekennzeichnet, dass** die Klemmhülse (2.4) so weit in den Ringspalt (1.3) eingeschoben wird, dass die Leitung (3) durch die Klemmhülse (2.4) in radialer Richtung um ein Maß zwischen 4 und 6 % einer ursprünglichen Wandstärke (3.3) deformiert wird.

9. Schlauchkupplung nach einem der Ansprüche 6 oder 7, **dadurch gekennzeichnet, dass** als Außengewinde ausgebildete Gewinde (2.2) in axialer Richtung an das Bedienteil (2.3) und an das Gewinde (2.2) in axialer Richtung die Klemmhülse (2.4) anschließt und das als Innengewinde ausgebildete Gewinde (1.7) am Gehäuseabschnitt (1.2) vorgesehen ist, wobei der Gehäuseabschnitt (1.2) in axialer Richtung über den Rohrstutzen (1.5) hervorsteht.

10. Schlauchkupplung nach einem der vorstehenden Ansprüche, **dadurch gekennzeichnet, dass** Bedienteil (2.3) und die Klemmhülse (2.4) einteilig und materialidentisch ausgebildet sind.

11. System bestehend aus einer Schlauchkupplung nach einem der vorhergehenden Ansprüche 1 bis 10 und einer Leitung (3).

12. Verfahren zum Anschließen einer Leitung (3) an eine zweiteilige Schlauchkupplung aus Kunststoff nach Anspruch 6 and 10, **gekennzeichnet durch** folgende Verfahrensschritte:
a) Auffädeln des Verschlusskörpers (2) auf die Leitung (3);
b) Einführen eines Endes (3.1) der Leitung (3) in den Ringspalt (1.3), bis eine Stirnfläche (3.2) eine Anschlagfläche (1.30) berührt, und gleichzeitiges Aufschieben des Endes (3.1) der Leitung (3) auf den Rohrstutzen (1.5);
c) Ein- oder Aufstecken des Verschlusskörpers (2) mit der Klemmhülse (2.4) in oder auf den Grundkörper (1), bis die beiden Gewinde (1.7, 2.2) sich berühren;
d) Verschrauben der beiden Gewinde, bis sich die beiden Anschlagflächen (1.6, 2.5) sichtbar berühren oder bis kein Spalt (4) mehr zwischen den Anschlagflächen (1.6, 2.5) erkennbar ist, wobei die Klemmkraft der Klemmhülse (2.4) kontinuierlich **durch** das Verschrauben bis zur Endlage E erhöht wird.

## Claims

1. A two-piece plastic hose coupling consisting of a base body (1) and a sealing body (2) for sealingly connecting a hose pipe or rigid line (3), wherein the base body (1) has
a) at least one channel (1.1) extending coaxially with respect to a center axis M in the base body (1) and running into a pipe socket (1.5) with a mouth (1.4), and
b) a housing portion (1.2) arranged coaxially with respect to the center axis M around the pipe socket (1.5), and an annular gap (1.3) formed between the channel (1.1) and the housing portion (1.2) and arranged coaxially with respect to the center axis M and being open toward the mouth (1.4), and the sealing body (2) has
c) a passage (2.1) for leading a line (3) through, said passage (2.1) being arranged coaxially with respect to the center axis M, and
d) an operating element (2.3) designed for manual screwing, wherein
e) the base body (1) and the sealing body (2) have at least one stop face (1.6, 2.5) each, said stop faces (1.6, 2.5) acting in a direction parallel to the center axis M, and the two stop faces (1.6, 2.5) adjoin each other indirectly or directly in a stop position E after connecting the hose pipe or rigid line (3), said adjoining being visible from the outside,
**characterized in that**
f) the sealing body (2) has a clamping element (2.4), which adjoins the passage (2.1) in the axial direction and can be inserted into the annular gap (1.3) in order to connect the line (3) and can be brought into contact with the line (3),
g) the clamping force of the clamping element is at its maximum value only when the base body (1) and the sealing body (2) are in the stop position E, said clamping element being in the form of a split taper socket (2.4), and
h) the split taper socket (2.4) forms a clamping face that is in the form of a line in the circumferential direction.

2. The hose coupling according to claim 1, **characterized in that** the housing portion (1.2) has a taper face (1.8), whose diameter increases toward the mouth (1.4) and which is directed toward the annular gap (1.3) and arranged coaxially with respect to the center axis M, the split taper socket (2.4) has a circumferential taper counterface (2.6), wherein an angle k of the taper face (1.8) is greater than an angle g of the taper counterface (2.6).

3. The hose coupling according to claim 2, **characterized in that** the depth of insertion (t) of the split taper socket (2.4) into the annular gap (1.3) has reached its maximum when the base body (1) and the sealing body (2) are in the stop position E.

4. The hose coupling according to any one of the preceding claims, **characterized in that** the two stop faces (1.6, 2.5) can be brought into direct contact with each other in the axial direction.

5. The hose coupling according to any one of the preceding claims, **characterized in that** the hose coupling is designed as a three-piece or multipiece hose coupling by means of a separate distance ring (5) and the two stop faces (1.6, 2.5) can be brought into indirect contact with each other in the axial direction via the distance ring (5).

6. The hose coupling according to any one of the preceding claims, **characterized in that** the base body (1) and the sealing body (2) have one thread (1.7, 2.2) each, said threads (1.7, 2.2) being arranged coaxially with respect to the center axis M, wherein the two threads (1.7, 2.2) can be screwed onto each other in order to connect the line (3).

7. The hose coupling according to claim 6, **characterized in that** the extent to which the split taper socket (2.4) can be inserted into the annular gap (1.3) in the axial direction by means of the two threads (1.7, 2.2) corresponds to the extent to which the base body (1) and the sealing body (2) are closed by means of the threads (1.7, 2.2) during the closing operation.

8. The hose coupling according to any one of the preceding claims, **characterized in that** the split taper socket (2.4) is inserted into the annular gap (1.3) to such an extent that the line (3) is deformed by the split taper socket (2.4) in the radial direction by between 4 and 6 % of an original wall thickness (3.3).

9. The hose coupling according to any one of claims 6 or 7, **characterized in that** the thread (2.2), which is designed as an external thread, adjoins the operating element (2.3) in the axial direction and the split taper socket (2.4) adjoins the thread (2.2) in the axial direction and the thread (1.7), which is designed as an internal thread, is provided at the housing portion (1.2), wherein the housing portion (1.2) projects over the pipe socket (1.5) in the axial direction.

10. The hose coupling according to any one of the preceding claims, **characterized in that** the operating element (2.3) and the split taper socket (2.4) are designed as one piece and made of the same material.

11. A system consisting of a hose coupling according to any one of the preceding claims 1 to 10 and a line (3).

12. A method for connecting a line (3) to a two-piece plastic hose coupling according to claims 6 and 10, **characterized by** the following procedure steps:
a) threading the sealing body (2) onto the line (3);
b) inserting one end (3.1) of the line (3) into the annular gap (1.3) until a front face (3.2) contacts a stop face (1.30), and simultaneously slipping the end (3.1) of the line (3) on the pipe socket (1.5);
c) putting the sealing body (2) with the split taper socket (2.4) in or on the base body (1) until the two threads (1.7, 2.2) contact each other;
d) screwing the two threads onto each other until the two stop faces (1.6, 2.5) visibly contact each other or until no gap (4) between the stop faces (1.6, 2.5) is visible any more, wherein the clamping force of the split taper socket (2.4) is continuously increased by the screwing operation until the stop position E is reached.

## Revendications

1. Raccord pour tuyau en deux parties en matière plastique, composé d'un corps de base (1) et d'un corps de fermeture (2) pour le raccordement étanche d'une conduite de tuyau ou de tube (3), le corps de base (1) présentant
a) au moins une gaine (1.1) qui est coaxiale à un axe médian M dans le corps de base (1) et se termine dans une tubulure (1.5) avec une embouchure (1.4) et
b) un tronçon de logement (1.2) disposé de façon coaxiale à l'axe médian M autour de la tubulure (1.5) et un interstice annulaire (1.3) formé entre la gaine (1.1) et le tronçon de logement (1.2), disposé de façon coaxiale à l'axe médian M et ouverte vers l'embouchure (1.4) et le corps de fermeture (2)
c) un orifice de passage (2.1) disposé de façon coaxiale à l'axe médian M pour le passage d'une conduite (3) et
d) une partie de manoeuvre (2.3) réalisée pour le vissage manuel,
e) le corps de base (1) et le corps de fermeture (2) présentant chacun au moins une face de butée (1.6, 2.5) agissant dans une direction parallèle par rapport à l'axe médian M, et les deux faces de butée (1.6, 2.5) étant, après le raccordement de la conduite de tuyau ou de tube (3), adjacentes l'une à l'autre dans une position extrême E indirectement ou directement de façon visible de l'extérieur,
**caractérisé en ce que**
f) le corps de fermeture (2) présente un élément de serrage (2.4) qui se raccorde à l'orifice de passage (2.1) dans la direction axiale et qui peut être introduit dans l'interstice annulaire (1.3) pour le raccordement de la conduite (3) et qui peut être posé contre la conduite (3),
g) la valeur de la force de serrage de l'élément de serrage réalisé en tant que douille de serrage (2.4) n'est maximale que quand le corps de base (1) et le corps de fermeture (2) se trouve dans la position extrême E, et
h) la douille de serrage (2.4) forme une surface de serrage linéaire dans la direction circonférentielle.

2. Raccord pour tuyau selon la revendication 1, **caractérisé en ce que** le tronçon de logement (1.2) présente une face de cône (1.8) dont le diamètre s'agrandit en direction de l'embouchure (1.4) et qui est dirigée dans l'interstice annulaire (1.3) et est disposée de façon coaxiale à l'axe médian M, **en ce que** la douille de serrage (2.4) présente une face de cône complémentaire (2.6) périphérique, un angle k de la face de cône (1.8) étant plus grand qu'un angle g de la face de cône complémentaire (2.6).

3. Raccord pour tuyau selon la revendication 2, **caractérisé en ce que** la profondeur d'enfichage (t) de la douille de serrage (2.4) dans l'interstice annulaire (1.3) est maximale quand le corps de base (1) et le corps de fermeture (2) se trouvent dans la position extrême E.

4. Raccord pour tuyau selon une des revendications précédentes, **caractérisé en ce que** les deux faces de butée (1.6, 2.5) peuvent être directement et réciproquement posées l'une contre l'autre dans la direction axiale.

5. Raccord pour tuyau selon une des revendications précédentes, **caractérisé en ce que** le raccord pour tuyau est réalisé en trois parties ou en plusieurs parties avec une bague d'espacement (5) séparée, et **en ce que** les deux faces de butée (1.6, 2.5) peuvent être indirectement adjacentes dans la direction axiale par le biais de la bague d'espacement (5).

6. Raccord pour tuyau selon une des revendications précédentes, **caractérisé en ce que** le corps de base (1) et le corps de fermeture (2) présentent respectivement un filet (1.7, 2.2) disposé de façon coaxiale à l'axe médian M, les deux filets (1.7, 2.2) pouvant être assemblés l'un à l'autre par vissage pour le raccordement de la conduite (3).

7. Raccord pour tuyau selon la revendication 6, **caractérisé en ce que** la douille de serrage (2.4) peut être introduite sur une certaine longueur dans la direction axiale dans l'interstice annulaire (1.3) par les deux filets (1.7, 2.2), sur laquelle longueur le corps de base (1) et le corps de fermeture (2) sont fermés par les filets (1.7, 2.2) lors de la rotation de fermeture.

8. Raccord pour tuyau selon une des revendications précédentes, **caractérisé en ce que** la douille de serrage (2.4) est poussée suffisamment loin dans l'interstice annulaire (1.3) pour que la conduite (3) soit déformée par la douille de serrage (2.4) dans la direction radiale sur une certaine longueur comprise entre 4 et 6 % d'une épaisseur de paroi (3.3) initiale.

9. Raccord pour tuyau selon une des revendications 6 ou 7, **caractérisé en ce qu'**un filet (2.2) réalisé en tant que filet extérieur se raccorde dans la direction axiale à la partie de manoeuvre (2.3), et **en ce que** la douille de serrage (2.4) se raccorde au filet (2.2) dans la direction axiale, et **en ce que** le filet (1.7) réalisé en tant que filet intérieur est prévu sur le tronçon de logement (1.2), le tronçon de logement (1.2) dépassant de la tubulure (1.5) dans la direction axiale.

10. Raccord pour tuyau selon une des revendications précédentes, **caractérisé en ce que** la partie de manoeuvre (2.3) et la douille de serrage (2.4) sont réalisées d'une seule pièce et avec un même matériau.

11. Système composé d'un raccord pour tuyau selon une des revendications précédentes 1 à 10 et d'une conduite (3).

12. Procédé de raccordement d'une conduite (3) à un raccord pour tuyau en deux parties en matière plastique selon les revendications 6 et 10, **caractérisé par** les étapes de procédé suivantes :
a) enfilement du corps de fermeture (2) sur la conduite (3) ;
b) introduction d'une extrémité (3.1) de la conduite (3) dans l'interstice annulaire (1.3) jusqu'à ce qu'une face frontale (3.2) touche une face de butée (1.30), et simultanément poussée de l'extrémité (3.1) de la conduite (3) sur la tubulure (1.5) ;
c) emboitement du corps de fermeture (2) avec la douille de serrage (2.4) dans ou sur le corps de base (1), jusqu'à ce que les deux filets (1.7, 2.2) se touchent ;
d) vissage des deux filets jusqu'à ce que les faces de butée (1.6, 2.5) se touchent visiblement ou jusqu'à ce qu'aucun interstice (4) ne puisse plus être distingué entre les faces de butée (1.6, 2.5), la force de serrage de la douille de serrage (2.4) étant augmentée en continu par le vissage jusqu'à la position extrême E.
